(19) Europäisches Patentamt
European Patent Office
Office européen des brevets

(11) Publication number: 0 269 351 A2

(12) EUROPEAN PATENT APPLICATION

(21) Application number: 87310132.3

(22) Date of filing: 17.11.87

(51) Int. Cl.4: C12P 7/64 , //(C12P7/64,C12R1:645,1:65,1:-785,1:845)

(30) Priority: 21.11.86 JP 279147/86

(43) Date of publication of application: 01.06.88 Bulletin 88/22

(84) Designated Contracting States: AT BE CH DE ES FR GB IT LI LU NL SE

(71) Applicant: LION CORPORATION
3-7, Honjo 1-chome
Sumida-ku Tokyo(JP)

(72) Inventor: Nishimura, Minoru
2-2-6-305, Izumi-cho
Narashino-shi Chiba(JP)
Inventor: Hasegawa, Masato
3-9-37, Kurosunadai
Chiba-shi Chiba(JP)
Inventor: Iwasaki, Ryozo
3-5-18-404, Takahama
Chiba-shi Chiba(JP)

(74) Representative: Arthur, Bryan Edward et al
Withers & Rogers 4 Dyer's Buildings Holborn
London EC1N 2JT(GB)

(54) **Method for producing fat containing gamma-linolenic acid.**

(57) A method for producing a fat containing γ-linolenic acid comprising the steps of culturing a microorganism belonging to the genus Absidia, the genus Mortierella , the genus Mucor, the genus Rhizopus or the genus Syncephalastrum with a fatty acid or an ester thereof as the carbon source, and converting the fatty acid or the ester thereof to γ-linolenic acid.

EP 0 269 351 A2

# METHOD FOR PRODUCING FAT CONTAINING γ-LINOLENIC ACID

## BACKGROUND OF THE INVENTION

### 1. Field of the Invention

The present invention relates to a method for producing a fat containing γ-linolenic acid by utilizing a microorganism

### 2. Description of the Related Art

Known in the art are the methods (1) - (5) shown below as a method for producing a fat containing γ-linolenic acid by utilizing microorganisms:

(1) A method in which mould fungi of the genus Mortierella is cultivated with a hydrocarbon as the carbon source, and a fat is recovered from the cultured mycelium (see: Japanese Unexamined Patent Publication (Kokai) No. 57-144986);

(2) A method in which mould fungi of the genus Mortierella is cultivated with a highly concentrated carbohydrate as the carbon source, and a fat is recovered from the cultured mycelium (see; Japanese Unexamined Patent Publication (Kokai) No. 60-168391);

(3) A method in which a fat producing microorganism of the genus Gilbertella is cultivated in a medium with a high carbon source concentration, and a fat is recovered from the cultured mycelium (see: Japanese Unexamined Patent Publication (Kokai) No. 61-37097);

(4) A method in which a fat producing microorganism of the genus (Thamnidium is cultivated in a medium with a high carbon source concentration, and a fat is recovered from the cultured mycelium (see: Japanese Unexamined Patent Publication (Kokai) No. 61-37096); and

(5) A method in which a fat producing microorganism of the genus Cunninghamella is cultivated in a medium with a high carbon source concentration, and a fat is recovered from the cultured mycelium (see: Japanese Unexamined Patent Publication (Kokai) No. 60-126091).

However, in all of the methods known in the art, cultivation is carried out with the use of hydrocarbons such as n-alkanes, carbohydrates such as glucose, or sodium acetate, as the carbon source, and a method using other substances has not been proposed. Accordingly, there is a demand for a method of cultivating a microoganism with other substances as the carbon source, and obtaining a fat containing γ-linolenic acid from the cultured product.

## SUMMARY OF THE INVENTION

Accordingly, the objects of the present invention are to meet the above-mentioned demand and to provide a method for producing fat containing γ-linolenic acid by utilizing substances other than hydrocarbons, carbohydrates, and sodium acetate as a carbon source.

Other objects and advantages of the present invention will be apparent from the following description.

In accordance with the present invention, there is provided a method for producing a fat containing γ-linolenic acid, which comprises culturing a microorganism belonging to the genus Absidia, the genus Mortierella, the genus Mucor , the genus Rhizopus or the genus Syncephalastrum with a fatty acid or the ester thereof as the carbon source, and converting the fatty acid or the ester thereof to γ-linolenic acid.

## DESCRIPTION OF THE PREFERRED EMBODIMENTS

The present inventors carried out various studies into the cultivation of microorganisms with various substances as the carbon source, and into obtaining fats containing γ-linolenic acid from the cultured products in view of the state of the art as described above, and consequently found that, when a microorganism belonging to the genus Absidia such as Absidia corymbifera, a microorganism belonging to the genus Mortierella such as Mortierellaisabellina, a microorganism belonging to the genus Mucor such as Mucor ambiguus, a microorganism belonging to the genus Rhizopus such as Rhizopus oryzae, or a microorganism belonging to the genus Syncephalastrum such as Syncephalastrum racemosum is cultivated with a fatty acid or the ester thereof as the carbon source, a growth equal to or more than that obtained when using, for example, a hydrocarbon or a carbohydrate as the carbon source, is exhibited, whereby the fatty acid or the ester thereof can be efficiently converted to γ-linolenic acid and, therefore, a fat with a high γ-linolenic acid content having a γ-linolenic acid content substantially equal to that in the case of a cultivation with the use of a hydrocarbon or a carbohydrate as the carbon source, can be obtained from the cultured product, to accomplish the present invention.

In the present invention, the microorganism as described above is grown with a fatty acid or the ester thereof other than γ-linolenic acid as the carbon source, while converting the fatty acid or the ester thereof to γ-linolenic acid, and therefore, by extracting the fat by a convenient means from the grown microorganism cells, a fat with a high γ-linolenic acid content can be obtained.

The present invention will now be described in more detail below.

The microorganisms of the genus Absidia , the genus Mortierella, the genus Mucor, the genus Rhizopus or the genus Syncephalastrum to be used in the present invention are not particularly limited in species, and any species belonging to these genera can be used. However, particularly, Absidia corymbifera (e.g., IFO 4010), Mortierella isabellina (e.g., IFO 7873), Rhizopus oryzae (e.g., IFO 5418), Syncephalastrum racemosum (e.g., IFO 4816) are suitably employed.

Also, the kind of fatty acid or the ester thereof used as the carbon source is not limited, but various kinds irrespective of whether they are saturated, unsaturated, and linear or branched, can be used. Preferably, fatty acids having 8 to 22 carbon atoms, particularly 8 to 18 carbon atoms, as exemplified by n-capric acid, n-caprylic aid, lauric acid, myristic acid, palmitic acid, stearic acid, oleic acid, linoleic acid, may be used. Also, as the ester, esters of these fatty acids with lower monovalent alcohols such as methanol, ethanol, propanol, or with polyhydric alcohols such as glycerine, are preferred. For example, palm oil constituted primarily of palmitic acid and oleic acid, soya oil, corn oil, peanut oil, ricebran oil constituted primarily of oleic acid and linoleic acid, rape seed oil constituted primarily of oleic acid, linoleic acid and ercinic acid, castor oil constituted primarily of oleic acid, and ricinoleic acid may be used.

These fatty acids and esters thereof may be used either alone or in any mixture thereof.

Further, in the present invention, desirably an adequate nitrogen source is provided during cultivation. In this case, although inorganic sources such as $(NH_4)_2SO_4$ and $NH_4NO_3$ can be used as the nitrogen source, organic nitrogen sources such as urea, peptone, soybean protein, and corn steep liquor, are more preferably used. In this case, the γ-linolenic acid content in the fat can be enhanced by making the weight ratio of the fatty acid or the ester thereof added as the carbon source, to the organic nitrogen source such as soybean protein, corn steep liquor, peptone, polypeptone, casamino acids, and dried yeast, within the range of 3:1 to 1:2.

According to the present invention, inorganic salts such as $KH_2PO_4$, $MgSO_4 \bullet 7H_2O$, $FeSO_4 \bullet 7H_2O$, $ZnSO_4 \bullet 7H_2O$, $CaCl_2 \bullet 2H_2O$, and $CuSO_4 \bullet 5H_2O$, or other growth factors such as yeast extract and malt extract, surfactants such as polyoxyethylene (60 mole) sorbitan fatty acid esters, and other nutrient sources also can be used during cultivation, if desired.

In the present invention, the cultivation method of the above microorganisms is not limited, but preferably the cultivation is carried out by, for example, shaking a culture or submerging a culture in a liquid medium, or by carrying out cultivation by a solid culture wherein a liquid medium is impregnated in, for example, a sponge. In this case, the above fatty acid or the ester thereof is preferably added in an amount of 10 to 100 g to one liter of the liquid medium, and the pH of the medium is preferably 3.5 to 5.5, the cultivation temperature is preferably 15 to 35°C, and the cultivation time is preferably about 5 to 10 days.

After completion of the cultivation, a known means can be used as the method for harvesting the fat containing γ-linolenic acid from the cultured product. For example, there may be suitably used the method in which, after collection of the microorganisms from the cultured product, the microorganism cells are disrupted by a homogenizer with the use of a disruption aid, simultaneously with extraction of the fat with an extracting solvent.

## Examples

The present invention will now be further illustrated by, but is by no means limited to, the following Examples, wherein all parts and percentages are expressed on a weight basis unless otherwise noted.

Example 1

A mixture of 30 g of palm oil, 3 g of soybean protein, 3 g of $KH_2PO_4$, 0.3 g of $MgSO_4 \bullet 7H_2O$, 0.2 g of yeast extract, 0.2 g of malt extract, 10 mg of $FeSO_4 \bullet 7H_2O$, 1.2 mg of $CaCl_2 \bullet 2H_2O$, 0.2 mg of $CuSO_4 \bullet 5H_2O$, and 1.0 mg of $ZnSO_4 \bullet 7H_2O$ mixed in one liter of distilled water was adjusted to pH of 4.5, and 50 ml of the mixture was then filled into a Sakaguchi flask having a volume of 500 ml. After the microoganism strain shown in Table 1 was transplanted into this medium, cultivation was carried out at a temperature of 30°C, a shaking stroke of about 7 cm, and a shaking number of 200/min. for 8 days. After the cultivation, microorganisms were collected with a glass fiber filter paper, and washed several times with distilled water and hexane, respectively, to remove the residual oil. The microorganism cells in the residual oil were disrupted and the fat was extracted by a homogenizer, with the use of chloroform/methanol = 2/1 (V/V) as the extracting solvent and glass beads as the distruption aid. The unsaponified product of the extracted fat was removed and then methyl-esterification was effected, followed by an analysis of the fatty acid composition by gas chromatography. The results are shown in Table 1.

Table 1

| Microorganism strain | IFO No. | D.C. (g/l) | T.L./D.C. (%) | γ/T.L. (%) |
|---|---|---|---|---|
| Absidia corymbifera | 4010 | 16.0 | 42 | 3.0 |
| Mucor ambiguus | 6742 | 14.2 | 35 | 3.6 |
| Mortierella isabellina | 7873 | 13.0 | 46 | 4.5 |
| " | 6739 | 14.0 | 38 | 3.8 |
| Rhizopus oryzae | 5418 | 20.6 | 36 | 4.0 |
| Syncephalastrum racemosum | 4816 | 17.2 | 43 | 4.6 |
| " | 4828 | 17.7 | 24 | 3.6 |

(Note) D.C. = Dry mycerium
T.L. = Total lipid in dry mycerium
γ = Amount of γ-linolenic acid product.

These abbreviations are also the same in Tables 2 - 4.

Example 2

The same medium was used as used in Example 1, except that the palm oil was replaced by soya oil, rice bran oil, rape seed oil, corn oil, castor oil or peanut oil as the carbon source, Mortierella isabellina (IFO 7873) was transplanted thereto, and the cultivation and analysis were conducted under the conditions shown in Example 1. The results are shown in Table 2.

Table 2

| Carbon source | D.C. (g/l) | T.L./D.C. (%) | γ/T.L. (%) |
|---|---|---|---|
| Soya oil | 14.3 | 35 | 3.3 |
| Ricebran oil | 18.5 | 40 | 3.8 |
| Rapeseed oil | 21.2 | 37 | 1.9 |
| Corn oil | 16.7 | 39 | 2.9 |
| Castor oil | 8.7 | 35 | 8.3 |
| Peanut oil | 11.1 | 50 | 2.3 |

Example 3

A mixture of 30 g of oleic acid, 3 g of soybean protein, 3 g of $KH_2PO_4$, 0.3 g of $MgSO_4 \bullet 7H_2O$, 0.2 g of yeast extract, 0.2 g of malt extract, 10 mg of $FeSO_4 \bullet 7H_2O$, 1.2 mg of $CaCl_2 \bullet 2H_2O$, 0.2 mg of $CuSO_4 \bullet 5H_2O$, and 1.0 mg of $ZnSO_4 \bullet 7H_2O$ mixed in one liter of distilled water was adjusted to a pH of 4.5, and 100 ml of the mixture was then filled into a Sakaguchi flask having a volume of 500 ml. After the microorganism strain shown in Table 3 was transplanted into this medium, cultivation was carried out at a temperature of 30°C, a shaking stroke of about 7 cm, and a shaking number of 200/min. for 8 days. After the cultivation, microorganisms were collected with a glass fiber filter paper, and washed several times with distilled water and hexane, respectively, to remove the residual oil. The microorganism cells in the residual oil were disrupted and the fat was extracted by a homogenizer, with the use of chloroform/methanol = 2/1 (V/V) as the extracting solvent and glass beads as the disruption aid. The unsaponified product of the extracted fat was removed and then converted to a methyl ester, followed by an analysis of the fatty acid composition by gas chromatography. The results are shown in Table 3.

Table 3

| Microorganism strain | IFO No. | D.C. (g/l) | T.L./D.C. (%) | γ/T.L. (%) |
|---|---|---|---|---|
| Absidia corymbifera | 4010 | 9.0 | 40 | 4.0 |
| Mucor ambiguus | 6742 | 7.2 | 29 | 4.6 |
| Mortierella isabellina | 7873 | 5.5 | 50 | 6.4 |
| " | 6739 | 6.4 | 62 | 5.8 |
| Rhizopus oryzae | 5418 | 18.6 | 30 | 4.2 |
| Syncephalastrum racemosum | 4816 | 10.4 | 54 | 3.0 |
| " | 4828 | 10.9 | 71 | 2.0 |

Example 4

A mixture of 30 g of a 9:1 mixture of methyl oleate and methyl linoleate, 3 g of soybean protein, 3 g of $KH_2PO_4$, 0.3 g of $MgSO_4 \bullet 7H_2O$, 0.2 g of yeast extract, 0.2 g of malt extract, 10 mg of $FeSO_4 \bullet 7H_2O$, 1.2 mg of $CaCl_2 \bullet 2H_2O$, 0.2 mg of $CuSO_4 \bullet 5H_2O$, and 1.0 mg of $ZnSO_4 \bullet 7H_2O$ mixed in one liter of distilled water was adjusted to a pH of 4.5, and 100 ml of the mixture was filled into a Sakaguchi flask having a volume of 500 ml. After the microorganism strain shown in Table 4 was transplanted into this medium, cultivation was carried out under the same conditions as in Example 1 for 8 days. After the cultivation, microorganisms were collected with a glass fiber filler paper, and washed several times with distilled water and hexane, respectively, to remove the residual oil. Then, the cultured product was analyzed according to the method as shown in Example 1 to obtain the results shown in Table 4.

Table 4

| Microorganism strain | IFO No. | D.C. (g/l) | T.L./D.C. (%) | γ/T.L. (%) |
|---|---|---|---|---|
| Mortierella isabellina | 7873 | 3.7 | 30 | 6.1 |
| Rhizopus oryzae | 5418 | 4.3 | 45 | 4.2 |

Example 5

A mixture of 10 g of palm oil, 3 g of corn steep liquor (abbreviated as C.S.L) (weight ratio = 10:3), 0.5 g of yeast extract, 0.5 g of malt extract, 3 g of $KH_2PO_4$ , 0.3 mg of $MgSO_4 \bullet 7H_2O$, 10 mg of $FeSO_4 \bullet 7H_2O$, 1.2 mg of $CaCl_2 \bullet 2H_2O$, 0.2 mg of $CuSO_4 \bullet 5H_2O$, and 1.0 mg of $ZnSO_4 \bullet 7H_2O$ mixed in one liter of distilled water was adjusted to a pH of 4.1. Also, for comparison, the same mixture of the above components except for using 10 g of palm oil and 1 g of corn steep liquor (weight ratio = 10:1) was prepared.

Then 50 ml each of the above mixtures were filled into a Sakaguchi flask having a volume of 500 ml. After the microorganism strain shown in Table 5 was transplanted into this medium, cultivation was carried out at a temperature of 30°C, a shaking stroke of about 7 cm, and a shaking number of 200/min. for 6 days. After the cultivation, microorganisms were collected with a glass fiber filter paper, and washed several times with distilled water and hexane, respectively, to remove the residual palm oil. The microorganism cells of the residual palm oil were disrupted and the fats extracted by a homogenizer, with the use of chloroform/methanol = 2/1 (V/V) as the extracting solvent and glass beads as the distruption aid. The unsaponified product of the extracted fat was removed and then converted to a methyl ester, followed by an analysis of the fatty acid composition by gas chromatography. The results are shown in Table 5.

Table 5

| | | Weight ratio (Palm oil:C.S.L. = 10:3) | | | Weight ratio (Palm oil:C.S.L. = 10:1) | | |
|---|---|---|---|---|---|---|---|
| Microorganism strain | IFO No. | D.C. (g/l) | T.L./D.C. (%) | γ/T.L. (%) | D.C. (g/l) | T.L./D.C. (%) | γ/T.L. (%) |
| Absidia corymbifera | 4010 | 7.5 | 17 | 6.9 | 9.4 | 23 | 3.0 |
| Mucor ambiguus | 6742 | 6.4 | 30 | 8.7 | 8.4 | 35 | 3.6 |
| Rhizopus oryzae | 5418 | 5.8 | 24 | 6.0 | 8.0 | 30 | 4.0 |
| Syncephalastrum racemosum | 4816 | 8.1 | 22 | 8.4 | 10.1 | 31 | 4.6 |
| " | 4828 | 8.8 | 18 | 6.7 | 10.4 | 24 | 3.6 |

C.S.L. = corn steep liquor

D.C. = Dry mycerium
T.L. = Total lipid in dry mycerium
γ = Amount of γ-linolenic acid product

As can be seen from Table 5, in all of the microorganisms strains, when the weight ratio of the carbon source (palm oil) to the organic nitrogen source (C.S.L.) is 10:3, a fat containing γ-linolenic acid with a concentration (γ/T.L.) 1.5-fold or more that in the case of a weight ratio of 10:1, is formed.

Example 6

The same medium as in Example 5 was prepared except that the amount of corn steep liquor charged was changed to 1 to 20 g (weight ratio = 10:1 to 10:20) in example 5. Then 50 ml of the mixture was filled into a Sakaguchi flask having a volume of 50 ml, and Mortierella isabellina (IFO 7873) was transplanted thereto. The culturing was carried out under the same conditions as in Example 5 and an analysis was made. The results are shown in Table 6.

Table 6

| Weight ratio (palm/C.S.L.) | D.C. (g/l) | T.L./D.C. (%) | γ/T.L. (%) |
|---|---|---|---|
| 10/1 | 6.5 | 45 | 4.5 |
| 10/3 | 8.0 | 46 | 6.6 |
| 10/5 | 7.4 | 30 | 9.4 |
| 10/10 | 9.9 | 30 | 9.1 |
| 10/15 | 10.8 | 28 | 8.9 |
| 10/20 | 11.9 | 26 | 7.4 |

(Note) * C.S.L. = corn steep liquor

It can be seen that fats with higher γ-linolenic acid contents can be obtained when the weight ratio of palm oil to corn steep liquor is 10/3 to 10/20, compared to when it is 10/1.

Example 7

A mixture of 50 g of palm oil, 15 g of soybean protein (weight ratio 10:3), 1.0 g of yeast extract, 1.0 g of malt extract, 10 g of $KH_2PO_4$ , 0.3 g of $MgSO_4 \bullet 7H_2O$, 10 mg of $FeSO_4 \bullet 7H_2O$, 1.2 mg of $CaCl_2 \bullet 2H_2O$, 0.2 mg of $CuSO_4 \bullet 7H_2O$, 1.2 mg of $ZnSO_4 \bullet 7H_2O$ mixed in one liter of distilled water was adjusted to a pH of 4.5. Two liters of this medium were placed into a 2-liter jar fermenter, and after Mortierella isabellina (IFO 7873) was transplanted, cultivation was carried out at a temperature of 30°C, under an aeration of 1 vvm, at a shaking rotational number of 400 rpm for 150 hours. After the cultivation, the product was treated in the same manner as in Example 5 to obtain 59 g of dried microorganism cells and 24 g of the total fat. The fatty acid composition of this product is shown in Table 7.

Table 7

| Fatty acid | 16:0 | 18:0 | 18:1 | 18:2 | 18:3 (γ) |
|---|---|---|---|---|---|
| (%) | 23.6 | 2.5 | 49.4 | 13.6 | 8.2 |

As described above, the method of the present invention can efficiently produce γ-linolenic acid from a fatty acid or the ester thereof, and therefore, according to the present invention, an oil or fat containing γ-linolenic acid with a high added value can be produced from inexpensive fatty acids or esters thereof harvested from animal and vegetable oils and fats.

**Claims**

1. A method for producing a fat containing γ-linolenic acid comprising the steps of:
culturing a microorganism belonging to the genus Absidia, the genus Mortierella, the genus Mucor, the genus Rhizopus or the genus Syncephalastrum with a fatty acid or an ester thereof as the carbon source, and
converting the fatty acid or the ester thereof to γ-linolenic acid.

2. A method as claimed in claim 1, wherein the microorganism belonging to the genus Absidia is Absidia corymbifera.

3. A method as claimed in claim 1, wherein the microorganism belonging to the genus Mortierella is Mortierella isabellina.

4. A method as claimed in claim 1, wherein the microorganism belonging to the genus Mucor is Mucor ambiguus.

5. A method as claimed in claim 1, wherein the microorganism belonging to the genus Rhizopus is Rhizopus oryzae.

6. A method as claimed in claim 1, wherein the microorganism belonging to the genus Syncephalastrum is Syncephalastrumracemosum .

7. A method as claimed in claim 1, wherein at least one component selected from the group consisting of n-capric acid, n-caprylic acid, lauric acid, myristic acid, palmitic acid, stearic acid, oleic acid, and linoleic acid and esters thereof is used as the fatty acid or the ester thereof.

8. A method as claimed in claim 7, wherein at least one component selected from the group consisting of palm oil, soya oil, ricebran oil, rape oil, corn oil, castor oil, and peanut oil is used as the fatty acid or the ester thereof.